# EUROPÄISCHE PATENTSCHRIFT

(11) **EP 0 803 226 B1**
(45) Veröffentlichungstag und Bekanntmachung des Hinweises auf die Patenterteilung: **10.03.2004**
(21) Anmeldenummer: 97106515.6
(22) Anmeldetag: 19.04.1997
(51) Int. Cl.: A61B 5/15

(54) **Blutentnahmevorrichtung mit einem eine Kanüle aufweisenden Halter**
Blood collection device with a handle having a hollow needle
Dispositif de prélèvement de sang avec un appui contenant une canule

(30) Priorität: 27.04.1996 DE 19617000
(43) Veröffentlichungstag der Anmeldung: 29.10.1997
(73) Patentinhaber: Sarstedt AG & Co., 51588 Nümbrecht (DE)
(72) Erfinder: Färber, Horst, 51588 Nümbrecht (DE)
(74) Vertreter: Valentin, Ekkehard, Dipl.-Ing.

(56) Entgegenhaltungen:
- EP-A- 0 051 399
- EP-A- 0 393 521
- EP-A- 0 678 279
- US-A- 4 758 231
- US-A- 5 423 758

## Beschreibung

Die Erfindung betrifft eine Blutentnahmevorrichtung mit einem Halter, der eine mit einer angeschärften Schneidkante ausgebildete, hohlnadelartige Kanüle aufweist, die von einem endseitig geschlossenen, elastischen, schlauchartigen Ventilgummi umhüllt ist, wobei der Halter mit einem das Ventilgummi von dessen Außenmantel her festlegenden Abschnitt versehen ist.

Aus der US-A 3 585 984 ist eine solche Blutentnahmevorrichtung mit einem Halter bekannt, der eine mit einer angeschärften Schneidkante ausgebildete, hohlnadelartige Kanüle aufweist. Die Kanüle ist von einem endseitig geschlossenen, elastischen, schlauchartigen Ventilgummi mit Luft zwischen der Kanüle und dem Ventilgummi umhüllt. Der Halter besitzt nach vorne hin einen domartigen Ansatz, in den ein mit dem Ventilgummi einstückiges, verdicktes Kopfstück bzw. Stopfen eintaucht. Dieses Kopfstück stellt damit quasi einen das Ventilgummi von dessen Außenmantel her festlegenden Abschnitt dar. Das Ventilgummi wird nämlich in einem vorderen Bereich, d.h. mit seinem verdickten Kopfstück formschlüssig auf die Kanüle aufgeschoben und wird demzufolge durch Reibungskräfte in seinem Inneren bezüglich der Kanüle und auf seinem Äußeren bezüglich des Halters festgelegt.

Bei einer durch die DE-C 18 12 742 bekanntgewordenen Blutentnahmevorrichtung wird ein einseitig offener, rohrenförmiger Halter verwendet, der eine an ihren beiden Enden mit einer angeschärften Schneidkante ausgebildete, hohlnadelartige Doppelkanüle aufweist deren hinteres Ende in den auch als Führungshülse dienenden Halter hineinragt. Während das hintere Kanülenende der doppelschneidigen Hohlnadel von dem elastischen Ventilgummi mit radialer Luft umschlossen wird, ragt das vordere Kanülenende aus der Stirnwand des Halters vor und dient zur Einführung in eine Vene eines Patienten. Das entnommene Blut gelangt in ein Blutentnahmeröhrchen, das zuvor von dem offenen Halterende her an den Halter angeschlossen worden ist. Beim Zusammenfügen von Halter und Blutentnahmeröhrchen durchdringt die Schneidkante des hinteren Kanülenendes zunächst die ihm zugewandte Wand bzw. den Boden des elastischen Ventilgummis und danach einen elastischen Verschlußstopfen des Blutentnahmeröhrchens, so daß letztendlich das hintere Kanülenende in das Blutentnahmeröhrchen hineinragt; das elastische Ventilgummi wird ziehharmonikaartig zusammengedrückt. Nach der Blutentnahme und dem Abziehen des Blutentnahmeröhrchensvon dem Halter nimmt das Ventilgummi selbsttätig wieder seine das hintere Kanülenende dichtend abschließende Ausgangslage ein. Der Halter könnte alternativ auch auf lediglich eine die Kanüle aufweisende Scheibe reduziert werden, d.h. die Führungshülse braucht nicht vorhanden zu sein. Die Halterscheibe ließe sich in einem solchen Fall mit dem Dom einer Kappe des Blutentnahmeröhrchens verbinden.

Eine andere Ausführung einer während der Blutentnahme mit einer Doppelkanüle ausgebildeten Blutentnahmevorrichtung ist durch das DE-U 80 16 927 bekanntgeworden. Die Doppelkanüle besteht dort aus einem einseitig offenen, zylindrischen Gehäuse mit einem einteilig angeformten Außenkonus und einer darin angeordneten einschneidigen, in situ das hintere Kanülenende bildenden Kanüle. Auf den Außenkonus wird ein Aufnahmekonus eines Halters mit einer endseitig angeschärften Kanüle bzw. Hohlnadel aufgesteckt, so daß nach dem Zusammenfügen dieser beiden Bauteile eine Doppelkanüle vorliegt, wie oben schon beschrieben. Mit dieser Nadel-Ausführung ist eine Adaption möglich, die es erlaubt, eine handelsübliche Luer-Kanüle mit einem Blutentnahmeröhrchen zu verbinden. Das auch hier vorhandene elastische Ventilgummi liegt der Hohlnadel des Halters bzw. Adapters eng an. Es liegt im Rahmen der Anwendungsmöglichkeiten, einen solchen Adapter mit Luer-Konus über einen speziellen Anschluß an einen z.B. mit Blut gefüllten Schlauch anzukoppeln.

Bei einer aus der DE-A 29 03 167 bekannten Sicherheits-Kanüle zur Mehrfach-Blutentnahme ist das elastische, sich beim Verbinden mit dem Blutentnahmeröhrchen ziehharmonikaartig zusammenschiebende Ventilgummi wiederum mit radialer Luft bzw. radialem Spiel zu dem hinteren Kanülenende der Doppelkanüle angeordnet, d.h. das Ventilgummi besitzt über seine gesamte Länge einen Außendurchmesser, der größer als der Außendurchmesser der Kanüle bzw. Hohlnadel ist.

Das bei den Blutentnahmevorrichtungen das hintere Kanülenende umschließende bzw. umhüllende Ventilgummi ermöglicht es, mehrere Blutentnahmeröhrchen durch Ankoppeln an die Doppelkanüle zu füllen. Bei einem eng an der Hohlnadel anliegenden Ventilgummi (vgl. DE-U 80 16 927) tritt zwischen der Nadel und dem Ventilgummi unvermeidlich eine Reibung auf, was dazu führt, daß das Ventilgummit aus der Ziehharmonika-Haltung zu langsam in die entspannte Ausgangsposition zurückschnellt; beim Wechseln der Blutentnahmeröhrchen ist in diesen Fällen daher häufig nicht zu vermeiden, daß Bluttropfen aus der Kanüle hervorquellen. Dies deshalb, weil das Ventilgummi das hintere Ende der angeschärften Doppelkanüle nicht schnell genug verschließen kann. Das wird dann vermieden, wenn das elastische, schlauchartige Ventilgummi einen radialen Abstand zu der Nadel einnimmt, d.h. diese mit Luft umhüllt (vgl. DE-A 29 03 167 und DE-C 18 12 742). Dadurch, daß das Ventilgummi nicht eng an der Nadel anliegt, ergeben sich allerdings Probleme, das Ventilgummi lagesicher in dem Halter bzw. Adapter vorzusehen. Das Ventilgummi der aus der DE-C 18 12 742 bekannten Blutentnahmevorrichtung ist deshalb an seinem offenen Ende mit einem Anschlagbund ausgebildet, der sich gegen eine Innenschulter des Halters anlegt und im übrigen in dieser Lage allein aufgrund der Reibung zwischen der Mantelfläche des Bundes und der Innenmantelfläche des Halters positioniert wird. Das Ventilgummi der durch die DE-A 29 03 167 bekanntgewordenen Doppelkanüle ist an seinem offenen hinteren Ende mit einem Außenbund ausgebildet, der in eine Ringnut der Stirnwand des Halters eingesetzt ist. Abgesehen davon, daß auch hier wiederum das Ventilgummi nur mit einem losen Sitz in dem Halter befestigt ist, bereitet zudem die Montage des Ventilgummis Schwierigkeiten und erfordert großes Geschick beim Einfädeln des elastischen Ventilgummis in die Ringnut.

Daneben sind zahlreiche andere Befestigungstechniken für das Ventilgummi bekannt, z.B. das formschlüssige Aufschieben des Ventilgummis auf einen zylindrischen Abschnitt im Mittelbereich der Kanüle (vgl. DE-A 37 40 269). Durch die EP-A 0 619 096 ist es bekanntgeworden, das Ventilgummi in seinem vorderen Bereich über eine kugelförmige Randwulst zu schieben, während demgegenüber die WO-A 95/16395 lehrt, das Ventilgummi über einen umlaufenden konusförmigen Bund mit Hinterschneidungen zu stülpen und dort gegebenenfalls noch zu verkleben. Gemäß der EP-A 0 678 279 wird das Ventilgummi mit einem umlaufenden Kragen in einen umlaufenden Rand im inneren des Halters eingehängt und durch einen Plastikeinsatz gesichert.

Der Erfindung liegt die Aufgabe zugrunde, eine Blutentnahmevorrichtung der eingangs genannten Art zu schaffen, die mit geringem Montageaufwand einen sicheren Sitz des Ventilgummis in dem Halter bzw. Adapter ermöglicht.

Diese Aufgabe wird erfindungsgemäß dadurch gelöst, daß der das Ventilgummi von seiner Außenseite her festlegende Abschnitt des Halters ein bleibend verformbares Klemmelement ist. Hiermit wird erreicht, daß das Ventilgummi nicht mehr lediglich lose bzw. reibschlüssig oder gar durch Verkleben in dem Halter angeordnet, sondern durch das erfindungsgemäße verformbare Klemmelement unverlierbar festgelegt ist; es kann nicht mehr von der Kanüle abgezogen werden und schon gar nicht mehr selbsttätig aus dem Halter herausfallen. Gleichwohl wird gewährleistet, daß zwischen dem Ventilgummi und der Nadel bzw. dem hinteren Kanülenende der Doppelkanüle über die gesamte Länge des Ventilgummis ein radialer Abstand gegeben ist, so daß zwischen diesen beiden Teilen keine Reibung entsteht und die Elastizität des Ventilgumis für einen sofortigen Verschluß des hinteren Kanülenendes sorgen kann, wenn ein Blutentnahmeröhrchen gewechselt wird; ein Blutaustritt an dieser Stelle wird somit vermieden. Die Herstellung des Ventilgummis erfordert keine übermäßigen Anforderungen an das Einhalten von Konturen und Toleranzen wie auch keine besonderen Abdichtungen auf dem Innenmantel des Ventilgummis erforderlich sind.

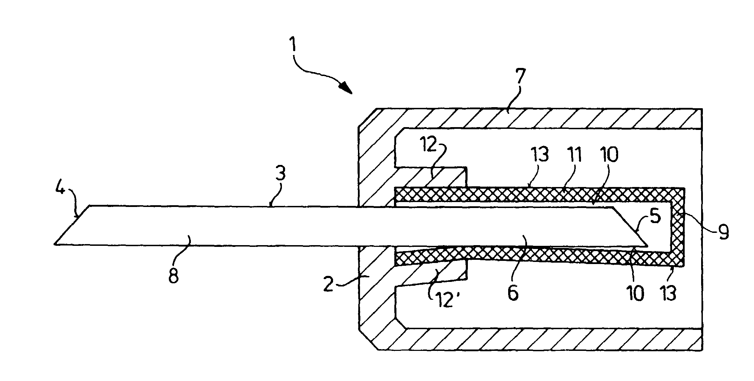

Weitere Merkmale und Vorteile der Erfindung ergeben sich aus der nachfolgenden Beschreibung, in der ein Ausführungsbeispiel des Gegenstandes der Erfindung anhand eines Halters für eine Blutentnahmevorrichtung näher erläutert ist, der als einseitig offener Halter mit einem röhrchenförmigen Abschnitt als Führungshülse ausgebildet ist und eine beidseitig angeschärfte Doppelkanüle aufweist. Gleichwohl sind alternativ die verschiedenen eingangs beschriebenen Halter-Ausführungen ebenfalls einsetzbar, wobei der Halter gegebenenfalls auch einstückig mit der Kanüle ausgebildet sein kann. Von einer als solche hinlänglich bekannten Blutentnahmevorrichtung ist in der einzigen Zeichnungsfigur ein einseitig offener Halter 1 gezeigt, in dessen Stirnwand 2 im AUsführungsbeispiel eine Doppelkanüle 3 durch z.B. Schweißen oder Kleben befestigt ist; auf einen zusätzlichen Nadelhalter kann daher verzichtet werden. Die Doppelkanüle 3 ist vorne und hinten mit einer Schneidkante 4 bzw. 5 ausgebildet; das hintere Kanülenende 6 ragt in die Führungshülse 7 des Halters 1 hinein, während das vordere Kanülenende 8 aus dem Halter 1 vorragt und zur Blutentnahme in eine Vene eines Patienten eingeführt wird. Zur Blutentnahme wird die Führungshülse 7 in bekannter Weise auf ein entsprechendes Blutentnahmeröhrchen aufgeschoben, wobei die Schneidkante 5 des hinteren Kanülenendes 6 sowohl den Boden 9 eines das hintere Kanülenende 6 mit radialem Abstand 10 umhüllenden Ventilgummis 11 als auch einen elastischen Verschlußstopfen des nicht dargestellten Blutentnahmeröhrchens durchdringt, bis die Verbindung zum Inneren des Blutentnahmeröhrchens hergestellt wird.

Das elastische, schlauchartige Ventilgummi 11 ist mit seinem von dem Boden 9 abgewandten offenen Ende in ein konzentrisch zu der Führungshülse 7 in dem Halter 1 an der Innenseite der Stirnwand 2 ausgebildetes Klemmelement 12 eingeschoben. Bei dem Klemmelement 12 kann es sich z.B. um einen umfangsgeschlossenen Ring oder einen Ring aus beabstandeten Teilsegmenten handeln, der bzw. dessen Ringsegmente nach dem Einstecken des Ventilgummis gegen den Außenmantel 13 des Ventilgummis 11 angestellt, dabei eingedrückt und zur Klemmung des Ventilgummis 11 bleibend verformt wird bzw. werden, wie in der unteren Zeichnungshälfte durch die Bezugsziffer 12' für das Klemmelement verdeutlicht. Das offene Ende des Ventilgummis 11 braucht somit lediglich bis zum Anschlag an die Innenseite der Stirnwand 2 in den Ringspalt zwischen dem Klemmelement 12 eingeschoben zu werden und liegt dann nach der Umformung eines oder mehrerer Klemmelemente sicher in dem Halter 1 fest.

Die Verformung des Klemmelementes oder einzelner Teil-Klemmelemente läßt sich beispielsweise durch einen Stempel erreichen, der eine Bohrung zur Aufnahme des hinteren Kanülenendes 6 einschließlich des diese umhüllenden, aufgesteckten Ventilgummis 11 aufweist. Wenn der vordere Abschnitt der Bohrung konisch geformt ist und einen größeren Durchmesser besitzt als das hülsenförmige Klemmelement 12 bzw. die zur Klemmung vorgesehenen einzelnen Klemmelemente, werden das bzw. die Klemmelemente bei einer axialen Stempelbewegung über den konischen Teil der Bohrung in den Außenmantel 13 des Ventilgummis 11 gedrückt (vgl. die Bezugsziffer 12' in der Figur). Nach dem Entfernen des Stempels verbleibt das Klemmelement 12 in dieser Position und klemmt das Ventilgummi 11 unverlierbar fest in dem Halter 1.

## Patentansprüche

1. Blutentnahmevorrichtung mit einem Halter (1), der eine mit einer angeschärften Schneidkante (4; 5) ausgebildete, hohlnadelartige Kanüle (3) aufweist, die von einem endseitig geschlossenen, elastischen, schlauchartigen Ventilgummi (11) mit Luft umhüllt ist, wobei der Halter (1) mit einem das Ventilgummi (11) von dessen Außenmantel her festlegenden Abschnitt versehen ist,
**dadurch gekennzeichnet,**
**daß** der das Ventilgummi (11) von seiner Außenseite her festlegende Abschnitt des Halters (1) ein bleibend verformbares Klemmelement (12) ist.

## Claims

1. A blood sampling device with a holder (1) which has a hollow-needle-type cannula (3) formed with a sharpened cutting edge (4; 5), said cannula being enveloped by an elastic, tube-like valve rubber (11) with air closed at the end, whereby the holder (1) is provided with a section fixing the valve rubber (11) from its outer sheath,
**characterised in that**
the section of the holder (1) fixing the valve rubber (11) from its outside is a permanently deformable clamping element (12).

## Revendications

1. Dispositif de prélèvement de sang comportant un support (1), qui présente une canule (3) en forme d'aiguille creuse conformée avec une arête coupante aiguisée (4 ; 5), qui est enveloppée d'air par un caoutchouc à vanne (11) élastique de forme tubulaire fermé côté extrémité, le support (1) étant pourvu d'une section positionnant le caoutchouc à vanne (11) à partir de sa chemise externe,
**caractérisé en ce que**
la section du support (1) positionnant le caoutchouc à vanne (11) à partir de sa chemise externe est un élément de blocage (12) durablement déformable.
